# EUROPEAN PATENT SPECIFICATION

(11) **EP 1 343 881 B1**
(45) Date of publication and mention of the grant of the patent: **14.03.2007**
(21) Application number: 01994731.6
(22) Date of filing: 27.11.2001
(51) Int. Cl.: C12N 15/09, C12N 1/20

(54) **METHOD FOR GENERATING COMPETENT OR TRANSFORMED CELLS**
VERFAHREN ZUR HERSTELLUNG VON KOMPETENTEN ODER TRANSFORMIERTEN ZELLEN
PROCEDE DE PRODUCTION DE CELLULES COMPETENTES OU TRANSFORMEES

(30) Priority: 21.12.2000 EP 00128049
(43) Date of publication of application: 17.09.2003
(73) Proprietor: Merck Patent GmbH, 64293 Darmstadt (DE)
(72) Inventor: RAYNER-BRANDES, Michael, D-64665 Alsbach (DE); WATKINS, Ian David, Morganstown, Cardiff CF15 8LT (GB)
(86) International application number: PCT/EP2001/013781
(87) International publication number: WO 2002/050252

(56) References cited:
- WO-A-97/28248
- DATABASE WPI Section Ch, Week 199412 Derwent Publications Ltd., London, GB; Class B04, AN 1994-094819 XP002207601 & JP 06 038740 A (NIPPON SEIHUN KK), 15 February 1994 (1994-02-15)

## Description

### Field of Invention

This invention relates to procedures for preparing competent cells from dried non-competent prokaryotic cells. The dried non-competent cells can be stored for long periods at a wide range of temperatures prior to use in these procedures. The invention also relates to procedures for simultaneously or subsequently transforming said competent cells.

### Background to the Invention

Competent cells possess the ability to be transformed, this means they have been treated to show an enhanced efficiency of uptake and establishment of exogenous DNA molecules; so that the genetic makeup of these cells is permanently changed. Cells that have not been specifically treated in this way show very low levels of uptake of extracellular DNA. Most typically, competent cells are used with procedures involving the uptake of cloning and expression vectors incorporating DNA sequences of particular interest to the user.

There are many procedures for preparing cells to be competent and for transformation of such cells. One of the first methods was described by Mandel and Higa (1970, Journal of Molecular Biology 53, 159) incorporating the steps of adding DNA to cells on ice in the presence of Ca²⁺ ions followed by a heat shock at 37-42 °C. A more established method for preparing competent cells, including the possibility to store the cells deep frozen, is to be found in the Laboratory Manual by Sambrook et al. (1989, Molecular Cloning, 2^{nd} Edition, 1.82). This describes a method whereby *Escherichia coli* cells are grown up in a suitable culture medium at 37 °C, then cooled on ice, separated from the medium by centrifugation and resuspended in ice-cold 0.1M CaCl₂. After addition of cryoprotectant the cell suspension can be quickly frozen and kept at -70 °C for subsequent use in transformation experiments. This particular Laboratory Manual describes a transformation method in which the cells, as prepared above, are thawed on ice, a small volume of DNA solution is added and mixed, the suspension is stored on ice for 30 minutes and then given a heat-pulse at 42 °C. After chilling on ice, a volume of culture medium is added, the cells are transferred to 37 °C to allow recovery and then the transformed cells are plated out to allow identification of transformants.

Sambrook et al. (1989) indicate a variety of chemicals which can be added to the solution in which the cells are suspended during the transformation procedure in order to aid the transformation process. These include: MES (2-[N-morpholino]ethanesulfonic acid), MnCl₂.4H₂O, KCl and Hexaaminecobalt chloride but many others are known to those skilled in the art.

Other methods used to make cells competent include, but are not limited to the following treatments:
- Inducing cells to contain high levels of poly-β-hydroxybutyrate/Ca²⁺/polyphosphate in a medium with at least one of the metal ions Ca²⁺, Mn²⁺, Sr²⁺ or Mg²⁺ (Huang, R & Rosetta, R.N., 1995, J. Bacteriol., 177, p486-490).
- Suspending cells in 0.1M CaCl₂ and polyethylene glycol at pH8.0 (Kurien, B.T. and Scofield, R.H., 1995, Biotechniques, 18, p1023-1026).
- Use of dimethylsulphoxide and dithiothreitol (Haiying Liu and Rashidbaigi Abbas, 1990, Biotechniques, 8, p.21-25).
- Use of phytic acid added to cells (Hara, N. et al, 1988, Nucleic Acids Research, 16, 8727).
- Use of sucrose, polyvinylpyrrolidone and EDTA (Taketo and Kuno, 1969, Journal of Biochemistry, 65, 369-373).

Changing the, growth conditions of the cells also has been found to influence competency:
- Using glycine in the growth medium improves transformation efficiency (Akhtar, M.K., Kaderbhai, N. and Kaderbhai, M.A., 1999, Analytical Biochemistry, 277, p273-276.
- Use of 10mM Mg²⁺ in the LB broth medium with glucose followed by storage at -80 °C in a solution containing 36% glycerol and 12% polyethylene glycol (Mw 7500) by Nishimura, A. et al., 1990, Nucleic Acids Research, 18, 6169.
- Presence of cycloserine in the medium (Norgard, M.V. et al., 1978, Gene, 3, 279-292).
- Deprivation of certain amino acids in the growth medium (Hauser, P.M. and Karamata, D., 1994, Microbiology, 140, 1613-1617).
- Growth in presence of 0.5M sucrose & addition of 1µg/ml lysozyme to DNA (Molholt, B. and Doskocil, J., 1978, Biochemical and Biophysical Research Communications, 82, 477-483).
- Another improved method for transforming E.coli is described in Patent No. US 4,981,797 by Jessee and Bloom (1991) whereby cells are grown at sub-optimal growth temperatures (<37 °C) before being made competent and then frozen.

Fast methods for making cells competent without heat shock have also been described:
- Cells not centrifuged: Nakata, Y., Xiaoren, T. and Yokohama, K., 1997, (Methods in Molecular Biology, 69, p129-137).
- Cells frozen to induce competence (Takashi, R. et al, 1992, Biotechniques, 13, 711-715).
- Chung, C.T. et al, 1989, (Proc. Natl. Acad. Sci. USA, 86, 2172-2175).
- Electrophoretic methods with growth in a salt-free medium: Sharma, R.C. & Schimke, R.T., 1996, (Biotechniques, 20, p42-44).

In all of the above described methodologies bacterial cells are grown, treated to enhance competency by varying complex procedures, then used directly or otherwise stored frozen at temperatures of -70°C or below. These storage requirements have a number of obvious disadvantages:-
- Shipment of such cells for commercial or other purposes conventionally requires the use of solid carbon dioxide within the packaging in order to sustain such low storage temperatures.
- Storage at temperatures of -70°C or below requires expensive and space-inefficient ultra cold freezer units.

Manufacture of commercial competent cells is a complicated, time-consuming process since methods for making cells competent on an industrial scale are very manpower intensive. Furthermore maintaining the viability and competency of the products based on these cells in the distribution chain to the customers requires a series of ultra-cold storage and transportation operations, with the attendant investment and revenue costs.

Improvements over these methodologies can be foreseen through procedures that simplify the preparation of competent cells and additionally allow the cells to be stored at temperatures at or above 0 °C prior to use in transformation experiments.

It is known that bacterial cells can be dried and sealed in containers to prevent ingress of water, thus allowing storage at room temperature or in a fridge for months or years. Heckly (1961, Advances in Applied Microbiology, 3, pp1-76) describes the successful application of freeze-drying and liquid-drying to the long-term preservation of bacteria. Drying from the liquid state as a cell preservation method, in this case the drying of *Salmonella ndolo* in suspensions to produce a foam, was also described by Annear, D.I., 1966 (Nature, 5050, 761). Such preservation techniques are essential to culture collections (for an overview see: Malik, K.A. & Claus, D., 1987, Biotechnology and Genetic Engineering Reviews, 5, pp 137-166) in which strains are preserved and maintained by freeze-drying and liquid-drying. Alternative drying strategies include, but are not limited to, convective drying (Lievense, L.C. and van't Riet, K., 1994, Advances in Biochemical Engineering/Biotechnology, 51, pp71-89), spray-drying (Franks and Hatley, 1992, EP0520748) and granulation of organisms (Müller, H., 1978, EP493761). Many other techniques are known by those expert in the art.

The only example of the stabilisation of competent cells by drying entails lyophilisation (Jessee and Bloom, 1997, Patent Application WO 98/35018) whereby the ice of frozen suspensions of competent cells in a freeze-drier is removed by sublimation under vacuum. This procedure is also very time consuming.

### Brief Description of the Invention

It has been discovered that cells dried for storage by such means as liquid-drying, freeze-drying or gas-drying, in all cases without prior treatment to induce a state of competency, can be made competent by reconstitution (rehydration) in aqueous solution of appropriate formulation. Furthermore, the cells can be transformed by additionally or subsequently adding DNA molecules to the solution containing these cells.

It is therefore even possible to directly pre-mix sample DNA in this reconstitution "competency" solution so that transformation can be initiated at the same time that the dried cells are being rehydrated and made competent.

Thus the method according to the present invention is quick and simple. The cells are stably stored in a substantially dry state without any special pre-treatment and can be rehydrated with competency solution when needed for transformation experiments. In particular, it is not necessary to dry and store competent cells as these may be more likely to lose viability in drying and storage than normal non-competent cells and therefore would typically require special storage conditions. It is an obvious point that survival of the dried cells is an important factor in successfully producing competent cells on rehydration with competency solution. It is sufficient to dry and store normal, non-competent cells and then treat them to make them competent by rehydrating them with competency solution directly prior to use.

A further benefit is that no thawing procedure is involved once the cells are removed from storage, in contrast to the situation when competent cells are stored frozen in solution.

The invention relates to a method for generating competent cells comprising
a) providing dried non-competent cells;
b) rehydrating said cells with competency solution in order to make them competent;

In a preferred embodiment of the present invention in step b) the cells are rehydrated with a competency solution containing calcium ions, preferably 0.1M CaCl₂ solution.

In another preferred embodiment the competency inducing solution is FSB Buffer (consisting of 20mM potassium acetate pH 7.5, 45mM MnCl₂.4H₂O, 4.8mM CaCl₂.2H₂O, 100mM KCl, 3mM hexamminecobalt chloride, 10% (v/v) glycerol made to pH 6.4 with 0.1N HCl) with or without 7% dimethylsulphoxide (DMSO) added. However, other similarly acting solutions may be used.

In another preferred embodiment the competency inducing solution is TB(DMSO). TB(DMSO) solution consists of 10mM HEPES, 15mM CaCl₂, 250mM KCl and 55mM MnCl₂ in AnalaR water. The pH of the buffer is adjusted to 6.7 using 0.25M KOH before the addition of MnCl₂. The solution is then filter sterilised using a 0.2µm filter and stored at +4°C. As required, 93mls of the buffer are aseptically transferred to a sterile container and 7ml DMSO added and mixed by inversion.

In a preferred embodiment the competency solution is formulated to neutralise contaminating compounds derived from the added DNA preparations which may be inhibitory or toxic to the competent cells.

In a preferred embodiment of the present invention, in step b) the cells are rehydrated with competency solution already containing DNA molecules to initiate transformation of the competent cells. This eliminates one step in the transformation procedures involving previously stored cells. For example, the equivalent stages with frozen competent cells are thawing of the stored cells, followed by addition of the DNA in solution. According to the present invention, the method of generating competent cells and optionally transforming them by the addition of DNA is called a microbiological procedure.

In an alternative embodiment of the present invention, if no DNA has been added with the competency solution, then subsequent to step b) in an additional step c) a solution containing DNA molecules is added to said mixture to initiate transformation of the competent cells.

In either case the procedures for transformation subsequent to addition of DNA which typically include incubation, heat-shock and other steps are well known to those skilled in the art.

In a preferred embodiment of the present invention, the DNA molecules added to the mixture at either stage b) or stage c) are the products of ligation or other in vitro annealing reactions involving the use of cloning or expression or other vectors to incorporate insert DNA sequences of particular interest, such as the products of polymerase chain reactions.

The present invention further relates to a kit for performing procedures according to the method of the present invention comprising dried non-competent cells and a competency solution or dried components for such a solution.

In a preferred embodiment the dried non-competent cells of the kit are provided in the wells of microtitre plates, such plates constructed as a single piece or separable into individual wells or even strips or rows of such wells.

In one embodiment the kit additionally comprises other components such as control DNA molecules, typically a suitable plasmid, and further optionally suitable vectors and other components for high-efficiency ligation and transformation.

### Description of the Drawings

*Figure 1* shows the transformation efficiency and viability of E. coli NovaBlue after drying and storage at +4°C
*Figure 2* shows the transformation efficiency and viability of E.coli NovaBlue after drying and storage at +20°C.
*Figure 3* shows the transformation efficiency and viability of E.coli JM109 after drying and storage at +4°C.
*Figure 4* shows the transformation efficiency and-viability of E.coli JM109 after drying, comparing liquid-dried and freeze-dried preparations.
*Figure 5* demonstrates the effect upon the transformation efficiency of E.coli JM109 and E.coli NovaBlue (after rehydration in competency solution) of pre-treating the cells to induce competency prior to drying, compared to cells dried without pre-treatment.
*Figure 6* shows the transformation efficiency of E.coli NovaBlue cells after drying and storage at +4°C, according to the method of Example 7.
*Figure 7* shows the transformation efficiency of E.coli NovaBlue cells after drying and storage at +4°C, according to the method of Example 8.
*Figure 8* shows the transformation efficiency of E.coli NovaBlue cells achievable in one experiment where all 5 flasks have been harvested, dried and tested separately, according to the method of Example 9.

Further description of the drawings can be found in the Examples of the Invention.

Abbreviations used in figures and elsewhere have the following meanings:
T/µg- Transformants per microgram of added DNA.
LB - Luria Broth
DMSO - Dimethylsulphoxide

### Detailed Description of the Invention

Both gram negative and gram positive prokaryotic cells (e.g. bacteria) can be used in accordance with the invention. Examples of suitable prokaryotic cells include but are not limited to, *Escherichia sp., Klebsiella sp., Staphylococcus sp., and Pseudomonas sp.* Non-limiting examples of species within each aforementioned genus that can be used in accordance with the invention include *Escherichia coli, Klebsiella sp., Bacillus subtilis, Salmonella typhimurium, Streptomyces aureus, Streptococcus mutans, Streptococcus pneumoniae, and Pseudomonas syringae.*

In a preferred embodiment, the cells which are rendered competent by the claimed method are *Escherichia,* most preferably *E.coli.* Non-limiting examples of *E.coli* strains which can be made competent by the claimed methods include E.coli, K12 strain "NovaBlue", DH5, DH5α, DH10, DH10B, HB101, RR1, JV30, DH11S, DM1, DH10B/p3, DH5α5'lQ, DH5α5',SCS1, Stab2, DH12S, DH5α-E, DH10BAC, XL1-Blue MRF, XL1-Blue MR, SURE Strain, SURE 2 Strain, XL1-Blue, XL2-Blue, AG1, JM101, JM109, JM110/SCS110, NM522, TOPP Strains, ABLE Strains, XL1-Red, BL21 Strains, TK BI Strain, and derivatives thereof.

According to the method of the invention, the cells to be made competent or transformed are cells that have been dried using known drying methods like freeze-drying, liquid-drying or gas-drying (including air-drying).

The drying methods are known to persons skilled in the art. Normally, the cells to be dried are grown in a growth conducive medium. Any medium capable of supporting growth of cells can be used. Examples of such media include but are not limited to Luria Broth (LB), LB supplemented with 20 mM MgCl₂, 0.001% thiamine, and 0.2% glucose; SOB medium and SOC medium (recipes given below) and 15/10 medium. Other suitable media will readily be recognised by one of skill in the art.

The incubation temperatures for growing the cells may vary from about 3 °C to about 42 °C. Preferably, the cells are grown at about 37 °C. As one of ordinary skill in the art will understand, growth conditions, culture age, and media used can affect the viability of cells during and after drying processes. Generally, cells harvested in the mid-late logarithmic or stationary phase are most resistant to drying.

Preferably, cells are grown in a shake flask although the culturing of cells by other means, for example in, but not limited to, fermenters, is well known by those skilled in the art. Shake flasks of any number of commercially available sizes and materials are suitable. Preferably, 1 L conical flasks with 100 mL of medium are used for the culture. The actual conditions employed will be dependent on the cells being grown, flask size, temperature of incubation and rate of shaking as determined appropriate by one skilled in the art. Typically cells are grown to an optical density (OD) at 600 nm of about 0.6 to 0.8 units. Cells may be cultured once or subcultured repeatedly to achieve a desired OD.

Harvesting of the cells may be performed by centrifugation although other techniques to collect the cells are known to those skilled in the art. Prior chilling of the cultures on ice and maintenance of temperatures around 4 °C are preferred during the harvesting process.

There are many protectants known to those expert in the art which may be used to aid the drying of the bacteria and minimise loss of viability. These include, but are not limited to, aqueous solutions of individual or mixtures of substances, e.g. carbohydrates (either natural or synthetic), including sugars and derivatives thereof and/or polymers (sugar and non-sugar based) and derivatives thereof and/or ectoines or other such compatible solutes and/or buffers and/or amino acids, peptides or proteins and derivatives thereof. Ideally, the T_{g} of the product should be well above room temperature and that the water content should lie below 5% w/w.

It is possible to add colour dyestuffs to the cells in the protectant mixture in order to ensure that the dried material can be seen clearly to assist observation of rehydration.

According to the present invention, rehydration of the dried stably stored cells is done with competency solution. Competency solution means any solution or buffer which can be added to cells to significantly increase transformation efficiency. The competency solution may also be formulated to include compounds to neutralise substances inhibitory or toxic to the competent cells, such as ionic detergents or phenol, that may inadvertently be added as contaminants present in DNA preparations.

Apart from the aforementioned 0.1M CaCl₂ or FSB buffer with or without added DMSO or TB(DMSO) solution with or without included DMSO there are many published procedures for making cells competent, such as suspending cells in 0.1M CaCl₂ solution containing polyethylene glycol at pH 8.0 or in solutions containing DMSO and/or dithiothreitol or in solutions containing EDTA with sucrose and polyvinylpyrrolidone or in solutions containing spermine and/or spermidine. Indeed, there are many such solutions known to those expert in the art that could be used as competency solutions. Typically these solutions are added ice cold to the dried cells at or near the temperature of ice and then incubated for a period of time for rehydration of the cells.

It is also possible to rehydrate the cells in two steps by first adding water or another aqueous solution suitable for optimal cell survival during rehydration and then directly adding the competency solution. According to the present invention, the term "rehydration or rehydrating cells with competency solution" means directly treating dried cells with competency solution or treating dried cells with water and directly with competency solution. Preferably, the dried cells are directly rehydrated with competency solution.

That means, to generate competent cells that can be further transformed according to known methods, the procedures according to the present invention comprise the following steps:
a) providing dried non-competent cells;
b) rehydrating said cells with competency solution, in order to make them competent.

The procedures of the present invention can also then be used to directly generate transformed cells.

### 1)

In one aspect of the invention, after rehydration of the dried cells with competency solution and thus making them competent, the DNA is added in solution. The DNA may be have been previously purified and resuspended in water or a suitable buffer or may be contained in reaction mixtures such as ligation mixes used for the formation of annealed DNA molecules incorporating sequences of interest into vectors such as cloning or expression vectors. The procedures for preparation of DNA for transformation of competent cells are well known to those skilled in the art. The competency solution may also be formulated to include compounds to neutralise substances inhibitory or toxic to the competent cells, that can derive from contaminating compounds such as ionic detergents or phenol present in the solution containing DNA.

The cells are typically incubated with the DNA for about 3 to 15 minutes at a temperature between 0 and 8 °C, preferably for 5 minutes on ice at 0°C. The procedures subsequent to incubation of the cells with the DNA which typically include heat-shock and other steps are well known to those skilled in the art.

As a consequence, the method comprises the following steps:
a) providing dried non-competent cells;
b) rehydrating said cells with competency solution;
c) adding DNA in solution to the mixture;
d) incubation of the mixture containing the DNA
e) further transformation procedures as well known to those skilled in the art.

### 2)

In a preferred aspect of the present invention, the DNA is directly added to the dried cells in the competency solution.

This further shortens the time that is needed to generate transformed cells out of dried non-competent cells. This shortened method comprises the following steps:
a) providing dried non-competent cells;
b) rehydrating said cells with competency solution containing DNA
c) incubation of the mixture containing the DNA
d) further transformation procedures as well known to those skilled in the art.

After adding competency solution containing DNA to the cells, the corresponding mixture is incubated to allow rehydration for a short period of time. The cells are incubated with the DNA for about 3 to 15 minutes at a temperature between 0 and 8 °C, preferably for 5 minutes in ice at 0°C. The procedures subsequent to incubation of the cells with the DNA which typically include heat-shock and other steps are well known to those skilled in the art.

The transformed cells obtained by the procedures according to the present invention may then be selected according to techniques well known in the art including, for example, selection for antibiotic resistance genes on the vector DNA molecule. After the transformed cells have been selected they may be cultured according to known procedures in a microbiological growth conducive medium.

Thus, the present invention offers a simple and quick method for producing competent or transformed cells. The starting material of this procedure, the non-competent dried cells, can be stably stored or transported then stored until they are needed e.g. in transformation experiments. It is possible to create competent or even transformed cells from dried cells within 10 to 15 minutes. It is not necessary to perform any costly and complex microbiological culture procedures prior to making the cells competent. The cells which have been made competent and that have been transformed according to the method of the present invention show transformation efficiency of at least 1x10⁵, preferably at least 1x10⁶, more preferably at least 1x10⁸ transformants per microgram of DNA (T/µg).

The present invention also facilitates the development of commercial kit products based on convenience formats, such as multi-test formats of 96 wells and above, without some of the complications of drying or freezing and thawing, storing and shipping treated competent cells in these formats.

Thus in a further aspect of the invention, a kit is provided for producing competent cells according to the method of the present invention. The test kit comprises at least non-competent dried cells and a competency solution. In addition, further aids like incubation containers, and/or a written protocol of how to produce competent cells etc. might be included. The competent cells generated with this kit can then be transformed according to known methods.

In another aspect of the invention, a kit is provided for producing transformed cells according to the method of the present invention. The test kit comprises at least non-competent dried cells, a competency solution and typically also a solution of control DNA molecules (typically a suitable plasmid). In addition, further aids like incubation containers or a written protocol of how to produce transformed cells etc. might be included.
Further kit options include the supply of suitable vectors, such as cloning or expression vectors and other components for high-efficiency ligation and transformation.

In a further aspect of the invention, the dried cells of the kits are provided in micro-centrifuge tubes sealed after drying whilst within the dry atmosphere of the drying apparatus.

In a further aspect of the invention, the dried cells of the kits are provided in a container with multiple dried pellets of the cells, each sufficient for at least one experiment. The container may also have a desiccant to maintain the dry state of the pellets.

In a further aspect of the invention, the dried cells of the kits are provided in the wells of microtitre plates, such as 96-well plates. The plates can either be constructed as a one-piece unit or separable into individual wells, or even strips or rows of such wells. The cells in the wells may have been dried and stored in the presence of a desiccant or the wells may be individually or collectively sealed.

In a further aspect of the invention, the dried cells of the kits are provided in glass vials with stoppers.

In a further aspect of the invention, the dried cells of the kits are provided in plastic vials in microtitre format with individual lids or other seals that prevent ingress of water.

The competency solution may be provided in liquid or as a dry formulation.

### Definitions

In the description that follows a number of terms used in recombinant DNA technology are utilised extensively. In order to provide a clear and consistent understanding of the specification and claims, including the scope to be given such terms, the following definitions are provided.

**DNA or DNA Molecule.** Any DNA molecule of any size, from any source, including DNA from viral, prokaryotic, and eukaryotic organisms.

The DNA molecule may be in any form, including, but not limited to, linear or circular, and single or double stranded. Non-limiting examples of DNA molecules include plasmids, vectors, cloning vectors and expression vectors.

**Cloning vector.** A plasmid, phage DNA, a cosmid, or other DNA molecule which is able to replicate automonously in a host cell, and which is characterised by one or more restriction endonuclease recognition sites that may be cut in a determinable fashion, or which contains one or more sites for homologous recombination, without loss of an essential biological function of the vector, and into which a DNA fragment may be spliced in order to bring about its replication and cloning. The cloning vector may further contain a marker suitable for use in the identification of cells transformed with the cloning vector. Markers, for example, provide tetracycline resistance or ampicillin resistance.

**Expression vector.** A vector similar to a cloning vector but which is capable of expressing a gene which has been cloned into it, after transformation into a host. The cloned gene is usually placed under the control of (i.e., operably linked to) certain control sequences such as promoter sequences.

**Stably stored.** Within the meaning of the present invention, cells which are "stably stored" are able to withstand storage for extended periods of time at a suitable temperature, without appreciably losing their viability. Suitable storage temperatures vary from about room temperature to about (minus) -180 °C. Preferably, the storage temperature ranges from about +30 °C to about -80 °C, more preferably from about +8 °C to about -20 °C. The storage period of time may range from about 0 days to about 450 days, preferably from about 240 days to about 450 days, although longer storage times may be used at temperatures of about -20 °C and below.

**Competent cells.** Prokaryotic cells, including Gram-positive and Gram-negative bacteria having the enhanced ability to take up and establish an exogenous DNA molecule. This uptake process is referred to as transformation.

**Substantially dry.** The state of the dried preparation which means that it will contain less than 10% water, preferably less than 5% water, more preferably, less than 2% residual moisture.

**Liquid-drying.** A process by which water is removed from cells suspended in liquid (typically containing high concentrations of carbohydrate or substances that protect the cells during drying) by which the cell suspension is exposed to a vacuum to achieve drying.

**Gas drying.** A process by which water is removed from cells suspended in liquid (typically containing high concentrations of carbohydrate or substances that protect the cells during drying) by which the cell suspension is exposed to a dry gas atmosphere, typically air, nitrogen or argon gas to achieve drying. The atmosphere can be kept dry by exposure to a very dry dessicant, for example silica gel, in a sealed chamber. With time the suspension dries to such an extent that the product becomes substantially dry.

**Freeze-Drying.** The process of freeze-drying / lyophilisation is drying from the frozen state whereby ice and/or moisture is removed from frozen cells by sublimation under vacuum at low, subzero temperatures (e.g. below -20 °C), typically yielding a cake.

**T_{g}.** The glass transition temperature, T_{g}, as a general rule can be defined as the temperature which yields a viscosity on the order of 10¹⁴ Pa s. At T_{g} the viscosity changes by several orders of magnitude over a narrow temperature range.

### Examples of the Invention

### Example 1

A seed stock of E.coli NovaBlue, a K12 derivative, (Novagen, Catalogue No.69009 -3) was prepared as follows: NovaBlue cells were streaked on an LB agar plate (10g/l Tryptone (peptone from casein - Merck KGaA., Art. No. 1.07213), 5g/l Yeast Extract (Merck KGaA., Art No. 1.03753), 10g/l NaCl, 15g/l Agar, pH adjusted to 7.5 with NaOH), + 12.5µg/ml Tetracycline, (Merck KGaA, Art No. 1.08189), and the plate incubated for 16 hours at 37°C. A single colony was inoculated into a 30ml Universal vial (Sterilin, Art. No.128A) containing 3ml SOB medium (20g/l Tryptone, 5g/l Yeast Extract, 0.5g/l NaCl, 0.186g/l KCl and 10ml/l 2M Mg²⁺ solution (1M MgSO_{4.}7H₂O, 1M MgCl₂.6H₂O) pH adjusted to 7.0 with NaOH or HCl as appropriate) supplemented with 12.5µg/mlTetracycline, and incubated at 37°C. The culture (primary culture) was grown to an OD₆₅₀ₙₘ of 0.41 (Novaspec II Visible Spectrophotometer), transferred to +4°C and stored overnight.

An aliquot of 1 ml of the primary culture was used to inoculate 50ml SOB medium supplemented with 12.5µg/ml Tetracycline in a 250ml conical flask (Pyrex, Art. No. 1130/14), and incubated at 37°C, with shaking at 250rpm. The OD₆₅₀ₙₘ was followed until the culture was at 0.71. The flask containing the culture was placed on ice for 10 minutes. An aliquot of 50ml FTB (0.98g/l potassium acetate, 8.9g/l MnCl₂.4H₂O, 1.47g/l CaCl₂.2H₂O, 0.8g/l Co(NH₃)₆Cl₃, 0.74g/l KCI, 100ml glycerol, pH adjusted to 6.4 with HCl), pre-chilled to +4°C, was added to the culture and the flask swirled to mix on ice for 10 minutes. The culture was dispensed in 1ml aliquots into pre-chilled cryovials (NUNC, Art. No. 3.68632), and transferred to - 70°C storage.

A vial of the above seed stock was thawed at room temperature and inverted several times to mix. An aliquot of 200µl seed culture was transferred to a 500ml conical flask (Pyrex, Art. No. 1140/10) containing 100ml LB broth (10g/l Tryptone, 5g/l Yeast Extract, 10g/l NaCl, pH adjusted to 7.5 with NaOH) supplemented with 12.5µl Tetracycline and incubated at 37°C with shaking at 180rpm. After approximately 16 hours the culture had reached an OD₆₀₀ₙₘ of 1.13. An aliquot of 2.5ml of this culture was used to inoculate a 1 L flask (Pyrex Art. No.1140/14) containing 100ml LB Broth + 12.5µg/ml Tetracycline. The flask was incubated at 37°C, with shaking at 250rpm for approximately 3.5 hours to an OD₆₀₀ₙₘ of 0.72. The contents of the flask were sub-divided in 50ml-aliquots into 2 pre-chilled, 50ml centrifuge tubes (Falcon, Art. No.352070). The centrifuge tubes containing the cells were placed immediately on ice for 10 minutes. The cells were collected by centrifugation in an 5804R Eppendorf centrifuge using rotor type F34-6-38 fitted with rotor adaptors (Eppendorf Art. No. 5804 775.007) at 4000rpm, 4°C for 10 minutes. The centrifuge, rotor and adaptors were pre-chilled to +4°C.

The supernatant was decanted and the tubes inverted for 1 minute to remove any remaining supernatant. The pellets were each re-suspended with 4ml Sucrose based protectant, (20mM Hepes, 7.5% sucrose, pH adjusted to 6.77 with NaOH), pre-chilled to +4°C, and transferred to a pre-chilled 15ml centrifuge tube (Falcon Art. No. 352096). The cells were collected by centrifugation at 4000rpm, 4°C for 10 minutes using pre-chilled rotor adapters (Eppendorf Art. No.5804 771.001). The supernatant was decanted and the tubes inverted for 1 minute to remove any remaining supernatant. The cells in each tube were resuspended in 550µl of the Sucrose based protectant. The contents of each tube were pooled.

The cells were dispensed in a cold room at +4°C into pre-chilled glass vials (Schott Glaskontor, Art. No. 04120366 supplied by Adelphi (tubes) Ltd. part reference VCD005) and stoppers (West Pharmaceutical Services, Art. No. 1067, formulation PH701/40 wine red chlorobutyl rubber, supplied by Adelphi (tubes) Ltd, part reference FDIA14) inserted to the midway position. The glass vials were then transferred to the shelves of a drier unit (Edwards Supermodulyo) pre-set to 20°C. The vacuum was drawn to 1.2 x 10⁻¹ to 9.8 x 10⁻²mbar. The vials remained in the vacuum chamber from the point of commencing the vacuum for 2.5 hours, after which the vials were sealed under vacuum. The vials were transferred to +4°C storage. The transformation efficiency and viable count were ascertained after intervals of storage up to 112 days.

To assay the cells for transformation efficiency and viable cell count the vials were removed from +4°C storage and placed on ice. The reagent was tested using two methods.

Method A. The cells were resuspended with 50µl 0.1M CaCl₂ solution containing a 1µl aliquot of the pUC19 plasmid at a concentration of 0.2ng/µl, (New England BioLabs, Order No.304-1S - diluted to 0.2ng/µl in 10mM Tris/ 1mM EDTA, pH adjusted to 8.0 with 1M HCl) and gently aspirated to ensure all cells were resuspended from the surface of the glass. Using a pipette, the resuspended cells were transferred to a pre-chilled, 1.5ml centrifuge tube (Eppendorf Safelock Art. No. 0030.120.086) and returned to ice for a 5 minute incubation period. A 1µl sample was removed for the viable cell count during the 5 minute incubation. The cells were then heat shocked in a 42°C water bath for 30 seconds, and immediately returned to the ice bath for 2 minutes. An aliquot of 250µl SOC medium (20g/l Tryptone, 5g/l Yeast Extract, 0.5g/l NaCl, 0.186g/l KCl, 20ml/l 1M glucose and 10ml/l 2M Mg²⁺ solution (1M MgSO₄, 1M MgCl_{2.}6H₂O) pH adjusted to 7.0 with NaOH or HCl as appropriate), at room temperature, was added to the cells on ice. The cells were gently mixed by aspiration and transferred to 37°C for 30 minutes. The 1µl sample was used to carry out the viable cell count by standard dilution in LB broth and plating the cells on LB agar. Colonies were counted after 24 hours incubation at 37°C.

Method B. The cells were resuspended with 50µl 0.1M CaCl₂ solution and gently aspirated to ensure all cells were resuspended from the surface of the glass. A rehydration period involving incubation on ice was allowed before transferring the cells to a pre-chilled, 1.5ml centrifuge tube. The cells were returned to ice and a 1µl aliquot of the pUC19 plasmid at a concentration of 0.2ng/µl added to the cells. A further 5 minute incubation period on ice was allowed. The cells were then heat shocked in a 42°C water bath for 30 seconds, and immediately returned to the ice bath for 2 minutes. An aliquot of 250µl SOC, at room temperature, was added to the cells on ice. The cells were gently mixed by aspiration, a 10µl aliquot removed for the viable cell count, and transferred to 37°C for 30 minutes. The 10µl sample was used to carry out the viable cell count by standard dilution in LB broth and plating the cells on LB agar. Colonies were counted after 24 hours incubation at 37°C. This method was carried out using a 0 minute, 15 minute and 75 minute rehydration period. Where a 0 minute rehydration period was allowed the test plasmid was added to the 0.1M CaCl₂ as described in Method A.

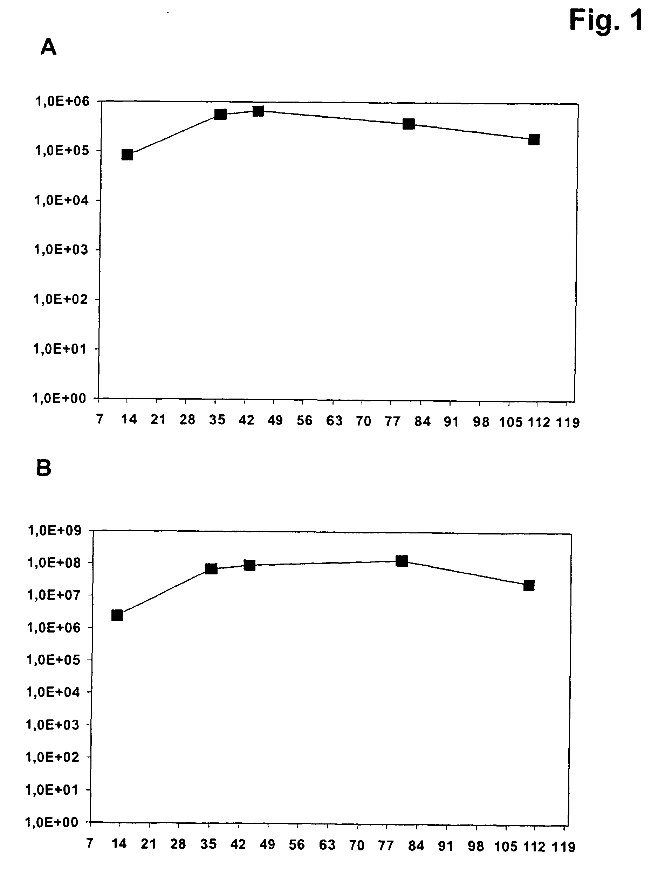

After 30 minutes incubation the cells prepared by either of Methods A and B were plated onto LB agar supplemented with 50µg/ml Carbenicillin, (Novagen, Art. No. 6910 1), 80µM Isopropyl-thio-β-D-galactopyranoside (IPTG), (Merck Ltd. Art. No. 437142L), and 70µg/ml 5-Bromo-4-chloro-3-indolyl-β-D-galactopyranoside (X-Gal), (Merck Ltd., Art. No.437132J). Colonies were counted after 18 hours incubation at 37°C. The results of the stability study associated with this experiment are presented in Figures 1A and 1B. (Fig 1A: x-axis: days of storage at 4°C, y-axis: T/µg; Fig 1B: x-axis: days of storage at 4°C, y-axis: cells/ml).

This trial at 4°C clearly demonstrated the long-term stability of the *E.coli* NovaBlue cells. The cells were dried for a short period of only two and a half hours at 20°C in a protectant based on sucrose. Both the transformation efficiency, Figure 1A, and the viable cell count, Figure 1B, remain above the values of the first test point for at least 15 weeks (at which point the trial was terminated). Table 1 illustrates that DNA can be added either directly to the rehydration solution or later, after the rehydration step has been performed - whichever is the more convenient.

**Table 1: Effect on transformation efficiency of duration of rehydration period before DNA addition:**

| Rehydration period | | |
|---|---|---|
| 0 mins* | 15 mins | 75 mins |
| 8.1E+04 T/µg | 1.1E+05 T/µg | 6.3E+05 T/µg |

| | | |
|---|---|---|
| *For 0 mins the DNA was added in the rehydration solution. Each result is expressed as the average of duplicates. | | |

### Example 2

The following experiment was carried out as for Example 1 with the following exceptions: The cells were harvested at an OD600ₙₘ of 0.8 units. After resuspension in protectant the cells were dispensed into pre-chilled 0.5 ml centrifuge tubes (Thermoquest Seg Ltd, Art No. PC1007) on ice. The stoppers of the tubes had been previously cut away. The lower end of each tube was then inserted into the internal bore of an inverted freeze drying bung (The West Company, 1121, W1816, Supplied by Laboratory Sales (UK) Ltd, part reference 700005) thus holding the tube vertically. A pre-chilled glass vial (Schott Amphabel 336040111, supplied by Adelphi (Tubes) Ltd, Order No. VC005-20C) was inverted, located over the centrifuge tube and pushed onto the inverted freeze drying bung to the mid-way position.

The cells in the centrifuge tube/glass vial configuration were then transferred to the shelves of a drier unit (Edwards Supermodulyo) pre-set to 20°C. The vacuum was drawn to 1.2 x 10⁻¹ to 9.8 x 10⁻² mbar. The vials remained in the vacuum chamber from the point of commencing the vacuum for 2.5 hours, after which the vials were sealed under vacuum by pressing the glass vial home on to the bung within the drier unit.

After drying, vials were placed at +4°C for 24 hours. The vials were then placed in sealable plastic bags and placed at +20°C. The transformation efficiency and viable count for the cells were ascertained after intervals of storage.

To assay the cells for transformation efficiency and viable cell count the vials were removed from storage and the centrifuge tubes removed and placed on ice. The cells were resuspended with 50µl 0.1M CaCl₂ solution containing a 1µl aliquot of the pUC19 plasmid at a concentration of 0.2ng/µl, and gently aspirated to ensure all cells were removed from the surface of the centrifuge tube. The cells were held on ice for a 5 minute incubation period. During this period a 1µl sample was removed to determine the viable cell count using standard dilution in LB broth and plating the cells on LB agar. Colonies were counted after 24 hours incubation at 37°C.

The cells were then heat shocked in a 42°C water bath for 30 seconds, and immediately returned to the ice bath for 2 minutes. An aliquot of 250µl SOC, at room temperature, was added to the cells on ice. The cells were gently mixed by aspiration and transferred to 37°C for 30 minutes.

After 30 minutes incubation the cells were plated onto LB agar supplemented with 50µg/ml Carbenicillin, 80µM lsopropyl-thio-β-D-galactopyranoside (IPTG), and 70µg/ml 5-Bromo-4-chloro-3-indolyl-β-D-galactopyranoside (X-Gal). Colonies were counted after 18 hours incubation at 37°C.

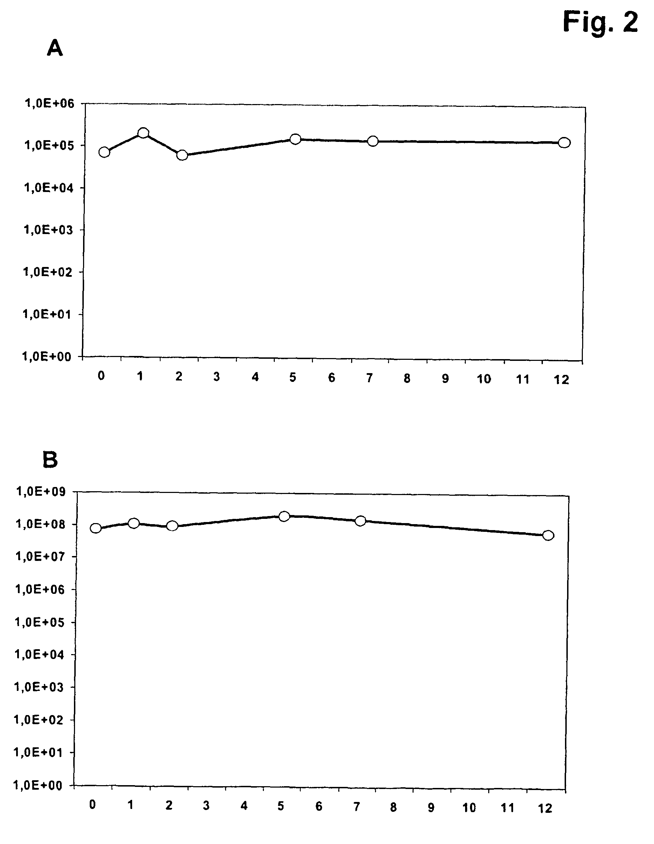

The transformation efficiency was stable over a period of at least 12 days at 20°C (at which point the trial was terminated) (see Fig. 2A). The viable count was stable over a period of at least 12 days at 20°C (see Fig. 2B). The results of the stability study associated with this experiment are presented in Figures 2A and 2B. (Fig 2A: x-axis: days of storage at 20°C, y-axis: T/µg; Fig 2B: x-axis: days of storage at 20°C, y-axis: cells/ml).

The stability trial demonstrated the stability of the viable count and the transformation efficiency of the *E.coli* NovaBlue cells at +20°C after liquid-drying for only two and a half hours in a protectant based on sucrose.

### Example 3

A seed stock of *E.coli* JM109, a K12 derivative, (DSMZ, DSM 3423) was prepared as follows: JM109 cells were streaked on an LB agar plate, and the plate was incubated for 72 hours at 20°C. A single colony was inoculated into a 30ml Universal vial containing 3ml SOC medium, and incubated at 37°C. The culture (primary culture) was grown to an OD₆₅₀ₙₘ of 0.40, transferred to +4°C, and stored overnight.

A 1ml aliquot of the primary culture was used to inoculate 50ml SOB medium in a 250ml conical flask and incubated at 37°C, with shaking at 250rpm. The OD₆₅₀ₙₘ was followed until the culture was at 0.65. The flask containing the culture was placed on ice for 10 minutes. An aliquot of 50ml FTB, pre-chilled to +4°C, was added to the culture and the flask swirled to mix on ice for 10 minutes. The culture was dispensed in 1ml aliquots into pre-chilled cryovials and transferred to - 70°C storage.

A vial of the above seed stock was thawed at room temperature and inverted several times to mix. An aliquot of 200µl seed culture was transferred to a 500ml conical flask containing 100ml LB broth and incubated at 37°C with shaking at 180rpm. After approximately 16 hours the culture had reached an OD₆₀₀ₙₘ of 1.33. An aliquot of 1ml of this culture was used to inoculate a 1L flask containing 100ml LB Broth. The flask was incubated at 37°C, shaking at 250rpm for approximately 3.5 hours to an OD₆₀₀ₙₘ of 0.8. The contents of the flask were sub-divided in 50ml aliquots into 2 pre-chilled, 50ml centrifuge tubes. The centrifuge tubes containing the cells were placed immediately on ice for 10 minutes. The cells were collected by centrifugation at 4000rpm, 4°C for 10 minutes. The centrifuge, rotor and rotor adapters were pre-chilled to +4°C.

The supernatant was decanted and the tubes inverted for 1 minute to remove any remaining supernatant. The pellets were each re-suspended with 4ml Sucrose based protectant, pre-chilled to +4°C, and transferred to a pre-chilled 15ml centrifuge tube. The cells were collected by centrifugation at 4000rpm, 4°C for 10 minutes using pre-chilled rotor adapters. The supernatant was decanted and the tubes inverted for 1 minute to remove any remaining supernatant. The cells in each tube were resuspended in 550µl of the Sucrose based protectant. The contents of each tube were pooled.

The cells were dispensed into pre-chilled 0.5ml centrifuge tubes (Treff AG, Treff Lab., Art No. 96.4625.9.01) on ice. The stoppers of the tubes had been previously cut away. The lower end of each tube was then inserted into the internal bore of an inverted freeze drying bung (The West Company, 1121, W1816, Supplied by Laboratory Sales (UK) Ltd, part reference 700005) thus holding the tube vertically. A pre-chilled glass vial (Schott Amphabel 336040111, supplied by Adelphi (Tubes) Ltd, Order No. VC005-20C) was inverted, located over the centrifuge tube and pushed onto the inverted freeze drying bung to the mid-way position.
The cells in the centrifuge tube/glass vial configuration were then transferred to the shelves of a drier unit (Edwards Supermodulyo) pre-set to 20°C. The vacuum was drawn to 1.2 x 10⁻¹ to 9.8 x 10⁻² mbar. The vials remained in the vacuum chamber from the point of commencing the vacuum for 2.5 hours, after which the vials were sealed under vacuum by pressing the glass vial home on to the bung within the drier unit.

After drying, vials of reagent were placed at +4°C for 24 hours. The vials were then placed in sealable plastic bags and placed at +4°C. The transformation efficiency and viable count for the cells were ascertained after intervals of storage.

To assay the cells for transformation efficiency and viable cell count the vials were removed from storage and the centrifuge tubes removed and placed on ice. The cells were resuspended with 50µl 0.1M CaCl₂ solution containing a 1µl aliquot of the pUC19 plasmid at a concentration of 0.2ng/µl, and gently aspirated to ensure all cells were removed from the surface of the centrifuge tube. The cells were held on ice for a 5 minute incubation period. During this period a 1µl sample was removed to determine the viable cell count using standard dilution in LB broth and plating the cells on LB agar. Colonies were counted after 24 hours incubation at 37°C.

The cells were then heat shocked in a 42°C water bath for 30 seconds, and immediately returned to the ice bath for 2 minutes. An aliquot of 250µl SOC, at room temperature, was added to the cells on ice. The cells were gently mixed by aspiration and transferred to 37°C for 30 minutes.

After 30 minutes incubation the cells were plated onto LB agar supplemented with 50µg/ml Carbenicillin, 80µM Isopropyl-thio-β-D-galactopyranoside (IPTG), and 70µg/ml 5-Bromo-4-chloro-3-indolyl-β-D-galactopyranoside (X-Gal). Colonies were counted after 18 hours incubation at 37°C.

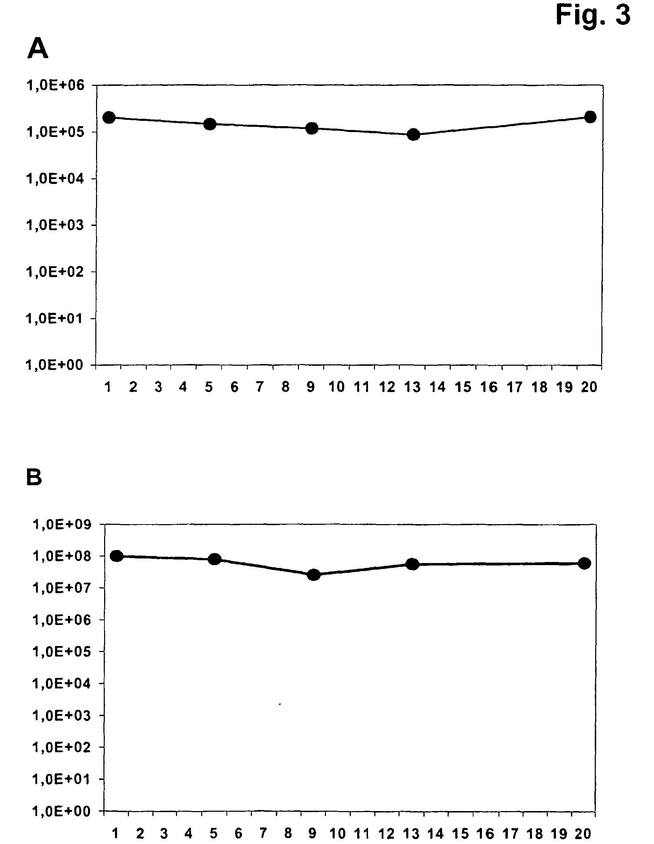

The transformation efficiency and viability were stable over a period of at least 20 days at 4°C (at which point the trial was terminated). The results of the stability study associated with this experiment are presented in Figures 3A and 3B. (Fig 3A: x-axis: days of storage at 4°C, y-axis: T/µl; Fig 3B: x-axis: days of storage at 4°C, y-axis: cells/ml).

The stability trial demonstrated the stability of the standard E.coli strain JM109 cells after liquid-drying for only two and a half hours in a protectant based on sucrose.

### Example 4

The following experiment was carried out using E.coli JM109. The protocol used for preparing these cells was as described for Example 3, with the following exceptions:
The dispensed vials were sub-divided into two sets. The first set (Liquid dried) were transferred to the shelves of a drier unit (VirTis Advantage 2.0 EL) pre-set to 20°C. The vacuum was drawn to 80mTorr, (1 x 10⁻¹ mbar). The vials remained in the vacuum chamber from the point of commencing the vacuum for 2 hours 50 minutes. The vials were sealed under vacuum and transferred to +4°C storage. The second (Freeze dried) set of vials were placed at -70°C for 3 hours prior to transferring to the shelves of the drier unit pre-set to -45°C. The vacuum was drawn to 80mTorr, (1 x 10⁻¹ mbar), before commencing the program outlined below.

| Temperature (°C) | Time (hours) |
|---|---|
| -45 | 4 |
| -35 | 2 |
| -25 | 2 |
| -15 | 2 |
| -5 | 2 |
| 5 | 2 |
| 20 | 2 |

| | |
|---|---|
| Note: Ramp rate set to 1 °C per minute. | |

These vials were also sealed under vacuum and transferred to +4°C storage for 24 hours. The transformation efficiency and viability were then ascertained according to the method described in Example 3.

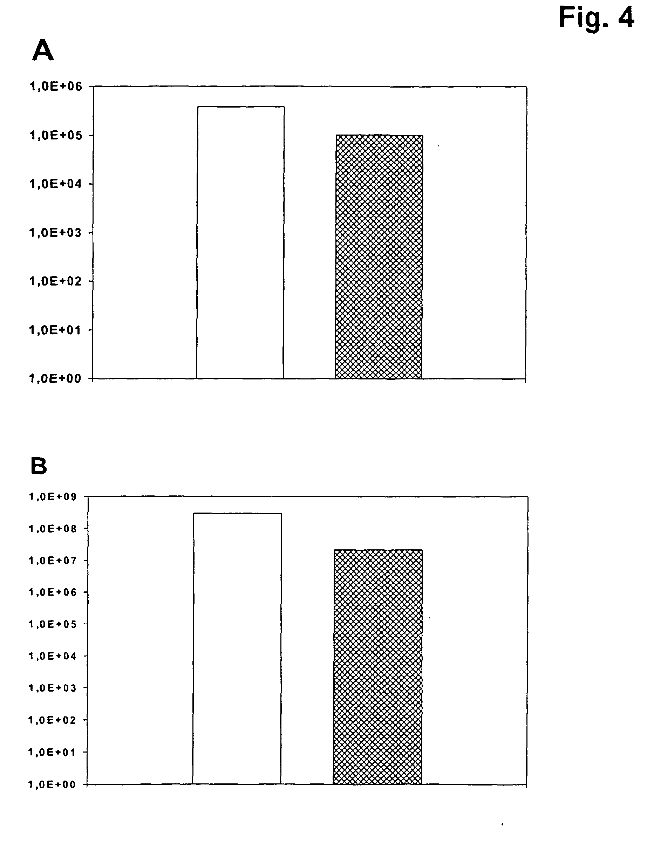

The results of the transformation efficiency and viability study associated with this experiment are presented in Figures 4A and 4B, respectively. (Fig 4A: y-axis: T/µg; Fig 4B: y-axis: cells/ml).

Figure 4A demonstrates that liquid-drying of cells results in similar transformation efficiency to freeze-drying the cells with the strain of E.coli tested. In general, liquid-drying is preferred to freeze-drying because of the greater simplicity of reagent preparation and the simpler drying equipment requirement.

### Example 5

The following experiment was carried out using E.coli NovaBlue and JM109. The protocols used for preparing these cells are as described for Example 2 and Example 3 respectively, with the following exceptions: After the first centrifugation step, one of the pellets was re-suspended with 15ml, pre-chilled, 0.1M CaCl₂ solution (pre-treated), the second pellet was re-suspended in 4ml sucrose based protectant only (not pre-treated). After resuspension, the cells were then centrifuged for a second time and each pellet was resuspended in 550µl sucrose based protectant. The contents of the centrifuge tubes were not pooled. The cells were dispensed into pre-chilled 0.5ml centrifuge tubes, (Treff AG, Treff Lab., Art No. 96.4625.9.01) in the arrangement as described in Examples 2 and 3. This procedure was carried out for both organisms.

The cells were transferred to the shelves of a drier unit (VirTis Advantage 2.0 EL) pre-set to 20°C. The vacuum was drawn to 80mTorr, (1 x 10⁻¹ mbar). The vials remained in the vacuum chamber from the point of commencing the vacuum for 2 hours 50 minutes. The vials were sealed under vacuum and transferred to +4°C storage.

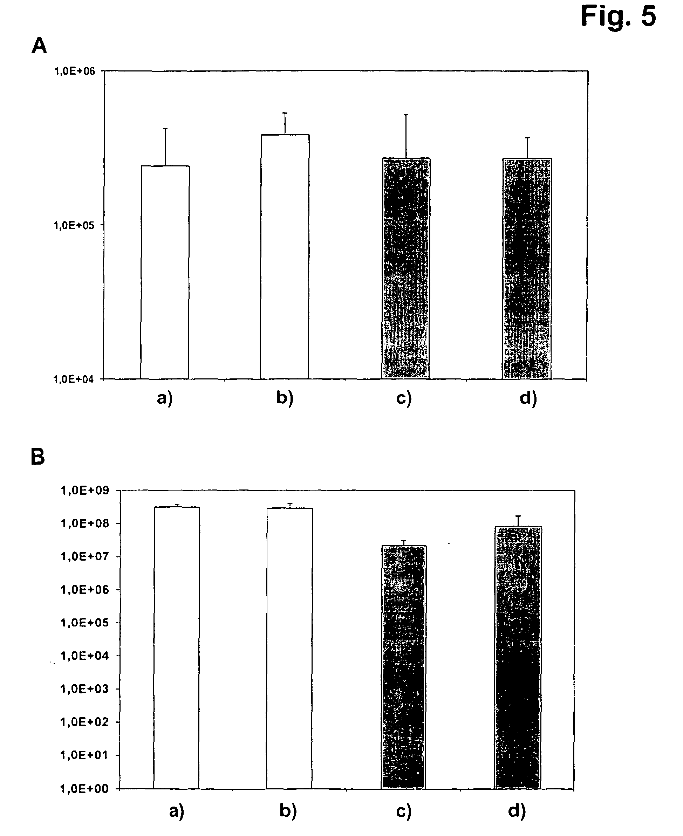

The results of the transformation efficiency associated with this experiment are presented in Figure 5. (Fig 5A: x-axis: a) JM109 pre-treated, b) JM109 not pre-treated, c) NovaBlue pretreated, d) NovaBlue not pre-treated, y-axis: T/µg; Fig 5B: x-axis: a) JM109 pre-treated, b) JM109 not pre-treated, c) NovaBlue pretreated, d) NovaBlue not pre-treated, y-axis: cells/ml).

Figure 5 demonstrates there is no significant difference in transformation efficiency between cells pre-treated with CaCl₂ to make them competent before drying, and cells dried without any such pretreatment but solely made competent by rehydration in competency solution. This result is not expected from the work described in WO 98/35018.

### Example 6

The following experiment was carried out using E.coli JM109. The protocols used for preparing these cells are as described in Example 3, with the following exceptions:
After the first centrifugation step, one of the pellets was re-suspended with 15ml, pre-chilled, 0.1M CaCl₂ solution (pre-treated), the second pellet was re-suspended in 4ml sucrose based protectant (not pre-treated). The cells were collected a second time by centrifugation and each pellet was resuspended in 550µl sucrose based protectant. The contents of the centrifuge tubes were not pooled. The cells were dispensed into pre-chilled 0.5ml centrifuge tubes, (Treff AG, Treff Lab., Art No. 96.4625.9.01) in the arrangement as described in Example 3.

The cells were transferred to the shelves of a drier unit (VirTis Advantage 2.0 EL) pre-set to 20°C. The vacuum was drawn to 80mTorr, (1 x 10⁻¹ mbar). The vials remained in the vacuum chamber from the point of commencing the vacuum for 2 hours 50 minutes.

The transformation efficiency was evaluated using either pre-chilled 0.1M CaCl₂ solution, or AnalaR Water [BDH Art. No. 102923C, Merck], or 0.9% NaCl solution to rehydrate the cells. An aliquot of 1µl of the pUC 19 plasmid, at a concentration of 0.2ng/µl, was added to the rehydration solution and the protocol followed as described in Example 3.

Table 6 clearly demonstrates that transformation efficiency of dried cells depends upon the treatment with competency solution during rehydration. Cells rehydrated with competency solution containing CaCl₂ gave very significantly higher transformation efficiency than those rehydrated instead, for example, in water or saline (0.9% NaCl). This is true whether or not the cells were pre-treated with calcium chloride to make them competent prior to drying. In other words, there is no benefit from drying competent cells, rather than non-competent cells in terms of resulting transformation efficiency. This result is not expected from the work described in WO 98/35018.

**Table 6: The effect of formulation of the rehydration solution on the transformation efficiency of CaCl₂ pre-treated and non pre-treated E.coli JM109 cells:**

| **Rehydration Solution** | **AnalaR Water** | | **0.1M CaCl₂** | | **0.9% NaCl** | |
|---|---|---|---|---|---|---|
| **CaCl₂ Pre-Treatment** | No | Yes | No | Yes | No | Yes |
| | | | | | | |
| **Transformation Efficiency [T/µg]** | 0 | 1E03 | 150E03 | 140E03 | 0 | 0 |
| **Viability [Cells/ml]** | 180E06 | 3E06 | 36E06 | 29E06 | 82E06 | 39E06 |

### Example 7

Aliquots (1ml) of frozen seed stocks of *E.coli* NovaBlue cells were prepared as in Example 1, thawed at room temperature and inverted several times to mix. The entire contents of one seed culture tube were transferred to each of two 2.8L fluted conical flasks (Fernbach type purchased through Philip Harris from Bellco, US, order no. 255102800) containing 550ml SOB medium (20g/l Tryptone, 5g/l Yeast Extract, 0.5g/l NaCl, 0.186g/l KCI) supplemented with 5ml of 2 molar Mg stock (1 molar MgCl₂.6H₂O from Fluka Art. no. 63068 and 1 molar MgSO₄.7H₂O from Fluka Art. no.63138) as well as 5ml of a 1 molar glucose solution. The culture was shaken at 275rpm. The addition of the supplements to SOB yields SOC. After approximately 24 hours both cultures had reached an OD₆₅₀ₙₘ of 0.51. Flasks were placed on ice for 15mins. The contents of the flasks were pooled and dispensed as two 245ml aliquots into pre-chilled, Nalgene 500ml centrifuge tubes (item no. 3140). The centrifuge tubes containing the cells were placed immediately on ice for approximately 10 minutes. The cells were collected by centrifugation in a Hereaus Sepatech centrifuge using rotor type 12.50 at 6000rpm, 4°C for 17 minutes. The centrifuge and rotor were pre-chilled to +4°C.

The supernatant was decanted and the tubes inverted for 1 minute to remove any remaining supernatant. The pellets were each re-suspended with 19.6ml Sucrose based protectant, (12% sucrose), pre-chilled to +4°C, and transferred to pre-chilled, 50ml centrifuge tubes (Falcon, Art. No.352070). The cells were collected by centrifugation at 6000rpm, 4°C for 12 minutes in a 5804R Eppendorf centrifuge using rotor type F34-6-38 fitted with rotor adapters (Eppendorf Art. No. 5804 775.007) at 5000rpm. The supernatant was decanted and the tubes inverted for 1 minute to remove any remaining supernatant. The cells in each tube were resuspended in 2.7ml of the Sucrose based protectant and pooled.

The cells were kept on ice and dispensed as 10µl aliquots into pre-chilled 1.5ml plastic centrifuge tubes (Thermoquest SEG Art. no. 02.1405.3500). Stoppers (West Pharmaceutical Services, Art. No.1067, formulation PH701/40 wine red chlorobutyl rubber, supplied by Adelphi (tubes) Ltd, part reference FDIA14) were inserted to the midway position after the tubes in aluminium racks were first transferred to solid carbon dioxide for at least 30 minutes. The tubes were then transferred to the shelves of a drier unit (Edwards Supermodulyo) pre-set to minus 45°C. The vacuum was drawn to 1.2 x 10⁻¹ to 9.8 x 10⁻² mbar. Drying was performed as in example 4 after which the tubes were sealed in situ under one atmosphere dry nitrogen gas.

The tubes were then taken and placed in plastic coated aluminium bags (Walter Coles Ltd art. no. 01085 / 1059533) in sets of 4 tubes per bag together with a silica gel sachet (Dryman art. no. 1059557). The bags were sealed with a Multivac Packager (a vacuum is drawn to about 10mbar, held for 2 seconds, flushed with dry nitrogen gas to 500mbar, held for 2 seconds, and sealed with pressure/heat held for 3 seconds before releasing to air). Bags were then stored at 4°C.

After storage at 4°C the bags were removed and cut open. The tubes were removed and placed on ice. The cells were rehydrated with 50µl TB(DMSO) solution and gently aspirated several times to resuspend cells (removal of stoppers immediately before rehydration).

TB(DMSO) solution was prepared by weighing out the HEPES (0.238g), CaCl₂.2H₂O (0.221 g), and KCl (1.864g) and dissolve in approximately 75ml AnalaR Water. Adjust the pH of the buffer to 6.7 using 0.25M KOH. Add the MnCl₂.4H₂O (1.088g) to the solution and dissolve. Transfer solution to a measuring cylinder and make up volume to 100ml using AnalaR Water. Filter sterilise using a 0.2µm filter and store at +4°C. Aseptically transfer 93ml of the buffer to a sterile container, add 7ml DMSO and invert to mix.

A 1µl aliquot of pUC19 plasmid at a concentration of 0.2ng/µl was added, and gently aspirated and flicked several times to ensure good mixing. The cells were held on ice for a 5 minute incubation period.

The cells were then heat shocked in a 42°C water bath for 30 seconds, and immediately returned to the ice bath for 2 minutes. An aliquot of 250µl SOC, at room temperature, was added to the cells on ice. The cells were gently mixed by aspiration and transferred to 37°C for 30 minutes

After 30 minutes incubation the tubes were taken and mixed by aspiration. Cells were plated onto LB agar supplemented with 50µg/ml Carbenicillin, 80µM Isopropyl-thio-*β*-D-galactopyranoside (IPTG), and 70µg/ml 5-Bromo-4-chloro-3-indolyl-β-D-galactopyranoside (X-Gal). Colonies were counted after 18 hours incubation at 37°C.

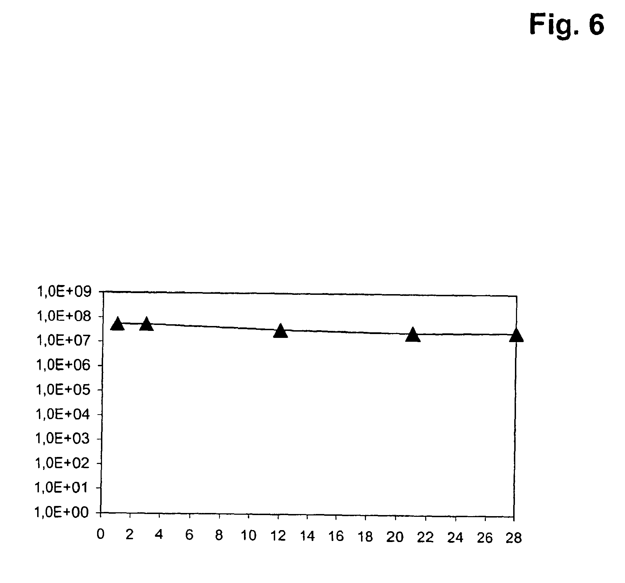

The results of the stability study associated with this experiment are presented in Figure 6. (x-axis: days of storage at +4°C, y-axis: T/µg).

### Example 8

Aliquots (1 ml) of frozen seed stocks of *E.coli* NovaBlue cells were prepared as in Example 1, thawed at room temperature and inverted several times to mix. The entire contents of one seed culture tube were transferred to each of two 2.8L fluted conical flasks (Fernbach) containing 550ml SOC and incubated with shaking at 275rpm. After approximately 22 hours both cultures had reached an OD₆₅₀ₙₘ of 0.54. Flasks were placed on ice for 15mins. The contents of the flasks were pooled and dispensed as two 400ml aliquots into pre-chilled, Nalgene 500ml centrifuge tubes (item no. 3140). The centrifuge tubes containing the cells were placed immediately on ice for approximately 10 minutes. The cells were collected by centrifugation in a Hereaus Sepatech centrifuge using rotor type 12.50 at 6000rpm, 4°C for 17 minutes. The centrifuge and rotor were pre-chilled to +4°C.

The supernatant was decanted and the tubes inverted for 1 minute to remove any remaining supernatant. The pellets were each re-suspended in 32ml sucrose based protectant, (12% sucrose), pre-chilled to +4°C, and transferred to a pre-chilled, 35ml polypropylene centrifuge tubes from Nalgene. The cells were collected by centrifugation at 6000rpm, 4°C for 12 minutes in the Hereaus centrifuge using rotor type 22,500. The supernatant was decanted and the tubes inverted for 1 minute to remove any remaining supernatant. The cells in each tube were resuspended in 4.4ml of the Sucrose based protectant and pooled.

The cells were kept on ice and dispensed as 10µl aliquots into pre-chilled 1.5ml plastic centrifuge tubes (Thermoquest SEG Art. no. 02.1405.3500). Stoppers (West Pharmaceutical Services) were inserted to the midway position before the tubes in aluminium racks were transferred to solid carbon dioxide for at least 30 minutes and then stored in a minus 70°C freezer. The tubes were then transferred to the shelves of a drier unit (Edwards Supermodulyo) pre-set to minus 45°C. The vacuum was drawn to 1.2 x 10⁻¹ to 9.8 x 10⁻² mbar. Drying was performed as in example 4 after which the tubes were sealed in situ under one atmosphere dry nitrogen gas.

The tubes were then taken and placed in sealed plastic coated aluminium bags for storage at 4°C as in example 6. At specified days bags were removed and reagent tested for transformation efficiency as in example 6.

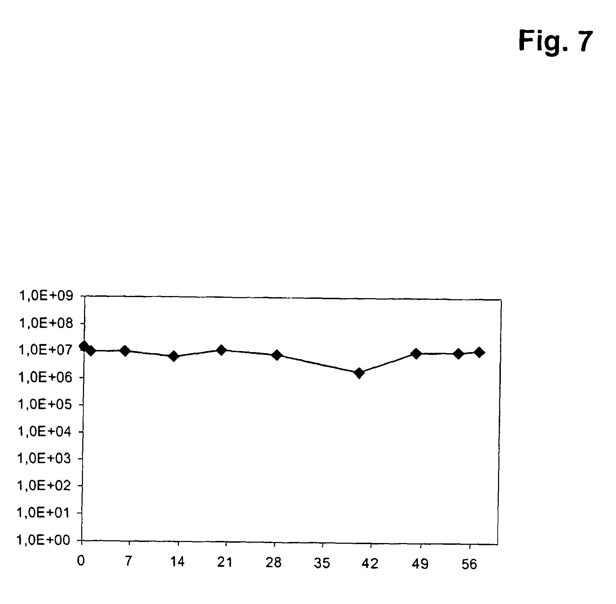

The results of the stability study associated with this experiment are presented in Figure 7. (x-axis: days of storage at +4°C, y-axis: T/µg).

### Example 9

Aliquots (1ml) of frozen seed stocks of *E.coli* NovaBlue cells were prepared as in Example 1. A vial of this seed stock was thawed at room temperature and inverted several times to mix. Aliquots of 50µl seed culture were transferred to each of five 250ml conical flasks (Duran, Art. No. 21-216-3) containing 25ml SOC medium supplemented with 163µl FTB solution and incubated with shaking at 275rpm. After approximately 24 hours the cultures had reached an OD₆₅₀ₙₘ of 0.59, 0.46, 0.48, 0.45 and 0.45 in the flasks respectively. The contents of the flasks were each poured into one of five pre-chilled, 50ml centrifuge tubes (Falcon, Art. No.352070). The centrifuge tubes containing the cells were placed immediately on ice for approximately 5 minutes. The cells were collected by centrifugation in an 5804R Eppendorf centrifuge using rotor type F34-6-38 fitted with rotor adaptors (Eppendorf Art. No. 5804 775.007) at 5000rpm, 4°C for 5 minutes. The centrifuge, rotor and adaptors were pre-chilled to +4°C.

The supernatant was decanted and the tubes inverted for 1 minute to remove any remaining supernatant. The pellets were each re-suspended with 2ml Sucrose based protectant, (12% sucrose), pre-chilled to +4°C, and transferred to a pre-chilled 50ml centrifuge tube (Falcon). The cells were collected by centrifugation at 5000rpm, 4°C for 5 minutes. The supernatant was decanted and the tubes inverted for 1 minute to remove any remaining supernatant. The cells in each tube were resuspended in 160µl of the Sucrose based protectant. The contents of each tube were treated separately.

The cells were kept on ice and dispensed as 10µl aliquots into pre-chilled 1.5ml plastic centrifuge tubes (Thermoquest). Stoppers (West Pharmaceutical Services, Art. No.1067, formulation PH701/40 wine red) were inserted to the midway position. The tubes in aluminium racks were first transferred to solid carbon dioxide for at least 30 minutes and then transferred to the shelves of a drier unit (Edwards Supermodulyo) pre-set to minus 45°C. The vacuum was drawn to 1.2 x 10⁻¹ to 9.8 x 10⁻² mbar. Drying was performed as in example 4 after which the tubes were sealed in situ in nitrogen gas at one atmosphere pressure. The tubes were then taken and tested for transformation.

To assay the cells for transformation efficiency the tubes were removed from the freeze drier and placed on ice. The cells were then tested as in Example 6.

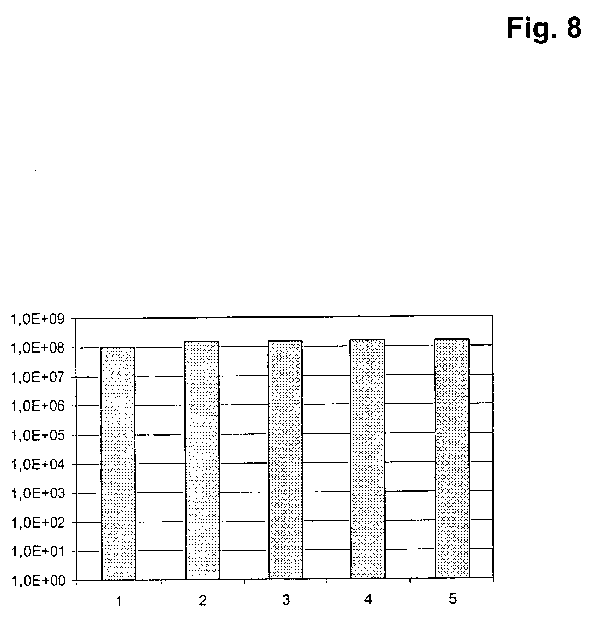

The results of the stability study associated with this experiment are presented in Figure 8. (x-axis: flask identification, y-axis: T/µg).

## Claims

1. Method for generating competent cells comprising
a) providing dried non-competent cells;
b) rehydrating said cells with competency solution

2. Method according to claim 1, **characterised in that** in step b) the cells are rehydrated with a competency solution containing calcium ions or FSB buffer with or without added DMSO or TB(DMSO) solution with or without included DMSO.

3. Method according to claim 1 or 2, **characterised in that** the competency solution is formulated to neutralise contaminating compounds derived from the added DNA preparations.

4. Method according to any of claim 1 to 3, **characterised in that** in step b) the cells are rehydrated with competency solution containing transforming DNA molecules.

5. Method according to any of claim 1 to 3 **characterised in that** subsequent to step b), the following additional step c) is performed:
c) adding the transforming DNA molecules to the mixture of step b).

6. Kit for performing the method of any of claims 1 to 5 comprising dried non-competent cells and a competency solution or dried components for such a solution.

7. Kit according to claim 6 **characterised in that** the non-competent dried cells are provided in the wells of microtitre plates.

## Patentansprüche

1. Verfahren zur Erzeugung von kompetenten Zellen, umfassend
a) die Bereitstellung von getrockneten nicht-kompetenten Zellen;
b) die Rehydration genannter Zellen mit Kompetenzlösung

2. Verfahren nach Anspruch 1, **dadurch gekennzeichnet, dass** in Schritt b) die Zellen mit einer Kompetenzlösung rehydriert werden, die Calciumionen oder FSB-Puffer mit oder ohne Zusatz von DMSO oder TB(DMSO)-Lösung mit oder ohne eingeschlossenem DMSO enthält.

3. Verfahren nach Anspruch 1 oder 2, **dadurch gekennzeichnet, dass** die Kompetenzlösung formuliert wird, um kontaminierende Verbindungen, die von den zugegebenen DNA-Präparaten abstammen, zu neutralisieren.

4. Verfahren nach einem der Ansprüche 1 bis 3, **dadurch gekennzeichnet, dass** in Schritt b) die Zellen mit einer Kompetenzlösung rehydriert werden, die transformierende DNA-Moleküle enthält.

5. Verfahren nach einem der Ansprüche 1 bis 3, **dadurch gekennzeichnet, dass** nachfolgend auf Schritt b) der folgende zusätzliche Schritt c) ausgeführt wird:
c) Zugabe der transformierenden DNA-Moleküle zu der Mischung aus Schritt b).

6. Kit für die Durchführung des Verfahrens nach einem der Ansprüche 1 bis 5, enthaltend getrocknete nicht-kompetente Zellen und eine Kompetenzlösung oder getrocknete Bestandteile für eine derartige Lösung.

7. Kit nach Anspruch 6, **dadurch gekennzeichnet, dass** die nicht-kompetenten getrockneten Zellen in den Löchern von Mikrotiterplatten bereitgestellt werden.

## Revendications

1. Procédé pour générer des cellules compétentes comprenant :
a) la fourniture de cellules non compétentes séchées ;
b) la réhydratation desdites cellules avec une solution de compétence.

2. Procédé selon la revendication 1, **caractérisé en ce que**, au niveau de l'étape b), les cellules sont réhydratées avec une solution de compétence qui contient des ions de calcium ou un tampon de FSB avec ou sans solution de DMSO ou de TB(DMSO) ajoutée avec ou sans DMSO inclus.

3. Procédé selon la revendication 1 ou 2, **caractérisé en ce que** la solution de compétence est formulée de manière à neutraliser des composés de contamination dérivés à partir des préparations d'ADN ajoutées.

4. Procédé selon l'une quelconque des revendications 1 à 3, **caractérisé en ce que** au niveau de l'étape b), les cellules sont réhydratées avec une solution de compétence qui contient des molécules d'ADN de transformation.

5. Procédé selon l'une quelconque des revendications 1 à 3, **caractérisé en ce que**, suite à l'étape b), l'étape additionnelle c) qui suit est réalisée :
c) ajout des molécules d'ADN de transformation au mélange de l'étape b).

6. Kit pour réaliser le procédé selon l'une quelconque des revendications 1 à 5, comprenant des cellules non compétentes séchées et une solution de compétence ou des composants séchés pour une telle solution.

7. Kit selon la revendication 6, **caractérisé en ce que** les cellules séchées non compétentes sont fournies dans les puits de plaques microtitre.
